# EUROPÄISCHE PATENTSCHRIFT

(11) **EP 2 716 259 B1**
(45) Veröffentlichungstag und Bekanntmachung des Hinweises auf die Patenterteilung: **14.12.2016**
(21) Anmeldenummer: 13004715.2
(22) Anmeldetag: 28.09.2013
(51) Int. Cl.: A61F 2/28

(54) **Prothese mit Nervaufnhame**
Prosthesis with nerve holder
Prothèse dotée d'un logement pour nerf

(30) Priorität: 06.10.2012 DE 202012009597 U
(43) Veröffentlichungstag der Anmeldung: 09.04.2014
(73) Patentinhaber: Schneider, Rosi, 76547 Sinzheim (DE)
(72) Erfinder: Schneider, Rosi, 76547 Sinzheim (DE)
(74) Vertreter: Wacker, Jost Oliver

(56) Entgegenhaltungen:
- WO-A1-2012/068062
- DE-A1- 4 226 465
- DE-A1-102005 041 412
- US-A- 4 636 215
- US-A1- 2010 215 718

## Beschreibung

Die Erfindung betrifft eine Prothese zum wenigstens teilweisen Ersatz eines Knochens, der einen Nerv führt, wie insbesondere eines Kieferknochens, nach dem Oberbegriff des Anspruchs 1. Die Prothese weist dabei an zwei voneinander abgewandten Enden Verbindungsmittel für die Festlegung der Prothese an verbleibenden Knochen- oder Gelenkteilen auf. Dabei ist eine Nervaufnahme für die Aufnahme des Nervs vorgesehen, die sich zwischen den beiden Enden der Prothese erstreckt.

In der heutigen Kieferprothetik ist es üblich, nach der operativen Entnahme eines Teils eines Kieferknochens, der beispielsweise von Krebs befallen ist, den fehlenden Kieferabschnitt mit einem Titangestell zu überbrücken. Hierzu werden beispielsweise beide Enden des Titangestells mit dem verbleibenden Kieferknochen verschraubt. Im Falle des Ersatzes eines Unterkieferteils ist es dabei üblich, den im sogenannten Mandibularkanal des Unterkiefers verlaufenden Nerv abzutrennen.

Nachteilig an dieser Vorgehensweise ist, dass der Patient hierbei sein Gefühl für Teile der Zähne und der Unterlippe verliert.

Aus DE 10 2005 041 412 A1 ist eine einstückige Teilprothese für einen Unterkiefer bekannt, die entweder geschlossen oder geschlitzt rohrförmig oder mit einem U-förmigen Querschnitt ausgebildet ist. Die Teilprothese weist dabei Durchgangslöcher 4 auf, über die sie mittels Schrauben oder Pins mit dem Unterkieferknochen verbunden werden kann.

WO 2012/068062 A1 beschreibt mehrere Ausführungsformen einer Prothese für einen Knochenabschnitt, der durch ein intraledullares Element überbrückt wird. Hierfür weist die Prothese in einer Ausführungsform zwei zueinander verschwenkbare Prothesenteile auf, in denen jeweils ein knochenaufbauendes Material aufgenommen werden kann. Dabei weisen die beiden Prothesenteile zwei aneinander anlegbare felxible Wandungen auf zwischen denen das intramedullare Element aufgenommen werden kann.

US 2010/0215718 A1 beschreibt eine schachtelförmige Aufnahme für knochenaufbauendes Material , die mittels einer Stabilisierungsplatte zwischen zwei erhaltene Kieferknochenabschnitte eingesetzt werden kann, um das knochenaufbauende Material in dem fehlenden Kieferknochenabschnitt zu ersetzen.

DE 42 26 456 A1, welche die Merkmale des Oberbegriffs des Anspruchs 1 offenbart, beschreibt eine retikuläre Schale aus einer Gitterstruktur, an der künstliche Wurzeln angeschraubt sind. US 4,636,215 zeigt eine aus nichtmetallischem Maschengewebe hergestellte Schale mit einem integrierten Gelenkkörper zum teilweisen Ersatz eines Kieferknochens.

WO 2013/034180 A1 beschreibt eine einstückige teilweise oder volle Unterkieferprothese mit einem innenliegenden Aufnahmekanal. Zur Herstellung der relativ komplexen Form der Prothese ist eine Additive-Manufacturing Technologie vorgesehen, wie beispielsweise Lasersintern vorgesehen.

Bei diesem Stand der Technik ist es möglich, den zu entfernenden Knochenabschnitt in einer Weise zu entfernen, bei der der Nerv über den betreffenden Abschnitt hinweg erhalten bleibt, um ihn dann innerhalb der Prothese geschützt aufnehmen zu können. Auf diese Weise bildet die Prothese nicht nur einen Ersatz für den Knochen, sondern beispielsweise im Falle eines Unterkieferknochens auch einen Ersatz für den Mandibularkanal, so dass der erhaltene Nerv weiterhin geschützt aufgenommen werden kann.

Die Aufgabe der Erfindung ist es, eine Prothese zur Verfügung zu stellen, mittels der der jeweilige Nerv einfacher aufgenommen und besser geschützt werden kann.

Diese Aufgabe wird durch eine Prothese mit den Merkmalen des Anspruchs 1 gelöst. Dabei ist die Nervaufnahme durch einen verschließbaren Aufnahmekanal gebildet, wodurch über die Länge der Prothese hinweg ein allseitiger Schutz des in der Nervaufnahme untergebrachten Nervs gewährleistet werden kann. Hierzu ist der verschließbare Aufnahmekanal wenigstens teilweise in einen ersten Prothesenteil eingelassen und durch einen zweiten Prothesenteil verschließbar. Hierdurch ist es besonders einfach möglich, den Nerv nach dessen Freilegung exakt im Aufnahmekanal unterzubringen, bevor dieser dann durch das zweite Prothesenteil verschlossen wird. Die Prothese kann auf diese Weise einfach angebracht werden, während gleichzeltig eine störungsfreie Aufnahme des Nervs gewährleistet werden kann. Zuderm ist es güngstig, wenn in eine Außenseite der Prothese wenigstens eine Implantataufnahme für die Festlegung eines Implantats eingelassen ist. Hierdurch können die Implantataufnahmen bereits vorgefertigt an der Prothese vorgesehen werden, um diese nach ihrer Befestigung am jeweiligen Knochen, wie insbesondere an einem Kieferknochen, einfach und in besonders stabiler Weise mit Implantaten versehen zu können. Die Ausbildung einer entsprechenden Prothese mit Implantataufnahmen zum wenigstens teilweisen Ersatz eines Knochens ist dabei, insbesondere an Knochenabschnitten, die keinen Nerv führen, auch ohne eine Nervaufnahme beziehungsweise auch bei einer einteiligen Ausführung möglich, wie beispielsweise an einem Oberkieferknochen.

Vorteilhafterweise ist der zweite Prothesenteil beweglich am ersten Prothesenteil gelagert, wodurch eine besonders einfache Anbringung der Prothese am verbleibenden Knochen möglich ist und ein exaktes Verschließen des Aufnahmekanals gewährleistet werden kann.

Dabei ist es besonders günstig, wenn der zweite Prothesenteil über ein scharnierartiges Gelenk am ersten Prothesenteil verschwenkbar gelagert ist, wodurch eine besonders stabile Führung des zweiten Prothesenteils sichergestellt werden kann.

Alternativ hierzu kann der zweite Prothesenteil separat zum ersten Prothesenteil ausgebildet sein und mit diesem verschraubt werden, um die ursprünglich zueinander losen Prothesenteile am Knochen aneinander festzulegen. Hierdurch kann die Prothese besonders einfach um den Nerv herum angebracht werden.

Vorteilhafterweise ist an wenigstens einem Ende der Prothese eine Aufnahmeöffnung zur Aufnahme eines Schraubmittels vorgesehen. Hierdurch kann die Prothese relativ einfach beispielweise mittels einer Schraube mit dem verbleibenden Knochen verbunden werden. Bevorzugterweise wird dabei auch der verbleibende Knochen vor der Befestigung der Prothese bearbeitet, um einen Abschnitt der Prothese, in den die Aufnahmeöffnung eingelassen ist, besser aufnehmen zu können.

Erfindungsgemäß ist in der Nervaufnahme ein viskoses Lagermittel, wie beispielsweise Gelatine, vorgesehen, mittels dem der hierin aufgenommene Nerv gegen Reizungen geschützt werden kann.

Ferner ist es günstig, wenn die Prothese wenigstens teilweise aus einem massiven Material gebildet ist, wodurch das entfernte Knochenmaterial in besonders stabiler Weise ersetzt werden kann.

Alternativ oder zusätzlich hierzu ist es günstig, wenn die Prothese wenigstens zum Teil aus einer Gitterstruktur gebildet ist, die mit einem knochenaufbauenden Material gefüllt ist, wodurch eine besonders gute Biokompatibilität der Prothese erzielt werden kann, die auch ein stabiles Verwachsen der Prothese mit dem verbleibenden Knochenmaterial ermöglicht.

In den Figuren ist eine beispielhafte Ausführungsform der Erfindung dargestellt. Es zeigen:
- Figur 1: eine Ansicht eines einen Nerv führenden Knochen mit einem zu ersetzenden Knochenabschnitt,
- Figur 2: eine Ansicht des Knochen gemäß Figur 2 mit frei gelegtem Nerv und einer an dem zu ersetzenden Knochenabschnitt einsetzbaren Prothese,
- Figur 3: eine vergrößerte Ansicht der Prothese in einem geöffneten Zustand,
- Figur 4: eine perspektivische Ansicht eines Unterkieferknochens mit einer daran angeordneten alternativen Ausführungsform der Prothese im getrennten Zustand und
- Figur 5: eine pertspektivische Ansicht der Prothese nach Fig. 4 im befestigten Zustand.

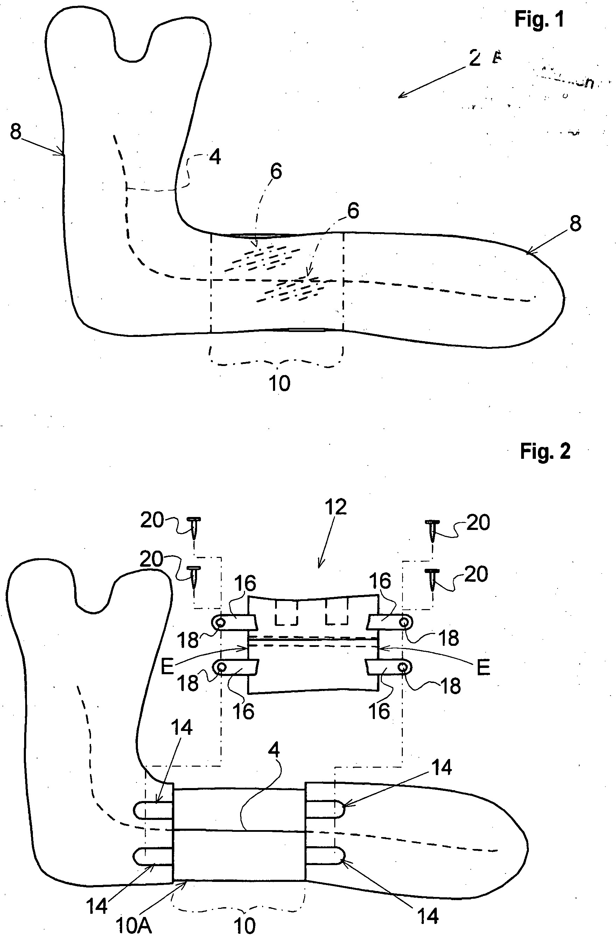

Fig. 1 zeigt einen Knochen 2, in dem ein Nerv 4 geführt ist und der beispielhaft durch einen Unterkieferknochen gebildet ist. Der Knochen 2 weist dabei erkrankte/zerstörte Bereiche 6 auf, die von den übrigen zu erhaltenden Knochen- oder Gelenkteilen 8 zu entfernen sind. Zu diesem Zweck wird ein Knochenabschnitt 10 bestimmt, der die Bereiche 6 mit umfasst und der im Zuge einer operativen Maßnahme entfernt wird.

Bei dieser operativen Maßnahme wird der Teil des Nervs 4, der sich über den Knochenabschnitt 10 hinweg erstreckt, gemäß Figur 2 weitestgehend frei gelegt und in jedem Fall nicht durchtrennt. Zu diesem Zweck wird vor dem Entfernen des Knochenabschnittes 10 die Lage des Nervs 4 beispielsweise mittels einer 3-dimensionalen digitalen Volumentomographie exakt bestimmt. Anschließend erfolgt das Entfernen beziehungsweise Abtragen des Knochenabschnittes 10 von wenigstens zwei Seiten des Nervs 4, um diesen vollständig erhalten zu können.

Nach dem Entfernen des Knochenabschnittes 10 wird das fehlende Knochenstück 10A beispielsweise wiederum mittels digitaler Volumentomographie exakt ausgemessen und analysiert. Mittels der hierbei gewonnenen Informationen wird dann eine bereits vorbereitete Prothese 12, die an Stelle des Knochenabschnittes 10 eingesetzt werden soll, gegebenenfalls angepasst und an den zu erhaltenden Knochen- oder Gelenkteilen 8 befestigt.

Hierzu können an den erhaltenen Knochen- oder Gelenkteilen 8 vorab Aufnahmekonturen 14 eingearbeitet worden sein, in denen Verbindungsmittel 16 der Prothese 12 besonders stabil gelagert werden können, die an zwei voneinander abgewandten Enden E der Prothese 12 vorgesehen sind. Die Verbindungsmittel 16 können dabei beispielsweise Aufnahmeöffnungen 18 aufweisen, durch die hindurch Schraubmittel 20 in die erhaltenen Knochen- oder Gelenkteile 8 einschraubbar sind, um die Prothese 12 zu befestigen.

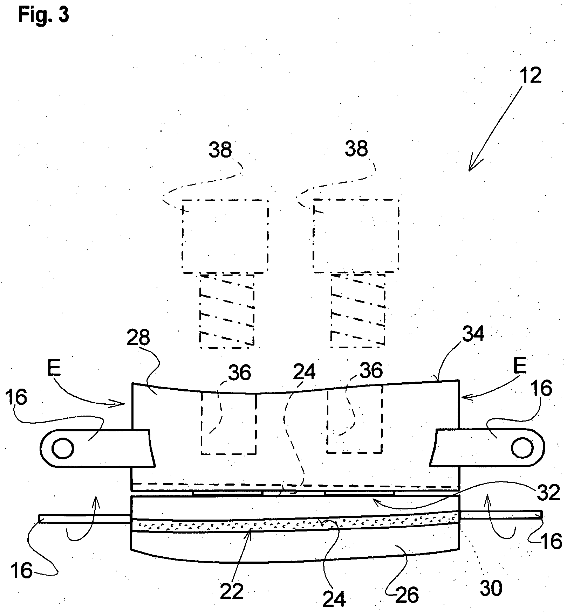

Um bei diesem Einbau auch den Nerv 4 in seinem weitestgehend vorgegebenen Verlauf problemlos und geschützt innerhalb der Prothese 12 aufnehmen zu können, weist diese, wie insbesondere aus Fig. 3 zu entnehmen ist, eine Nervaufnahme 22 auf, die sich zwischen beiden Enden E erstreckt. Die Nervaufnahme 22 ist dabei durch einen Aufnahmekanal 24 gebildet, der durch einen ersten Prothesenteil 26 und einen zweiten Prothesenteil 28 begrenzt ist, die zueinander beweglich gehalten sind. Je nach vorgegebenem Verlauf des Nervs 4 kann der Aufnahmekanal 24 dabei beispielsweise überwiegend in den ersten Prothesenteil 26 eingelassen sein, während der zweite Prothesenteil 28 im Wesentlichen dazu dient, den Aufnahmekanal 24 zu verschließen. Gegebenenfalls kann der Aufnahmekanal 24 aber auch teilweise in den ersten Prothesenteil 26 und teilweise in den zweiten Prothesenteil 28 eingelassen sein, wie in Fig. 3 dargestellt.

In jedem Fall ist in dem Aufnahmekanal 24 ein viskoses Lagermittel 30, wie beispielsweise Gelatine, vorgesehen, mittels dem der Nerv 4 vor Reizen und Verletzungen geschützt werden kann.

Wie aus Fig. 3 ferner zu entnehmen ist, können die beiden Prothesenteile 26, 28 zur leichteren Einführung des Nervs 4 in den Aufnahmekanal 24 und zum anschließenden Schließen derselben über ein scharnierartiges Gelenk 32 aneinander verschwenkbar gehalten sein.

Um die Prothese 12 anschließend an den verbleibenden Knochen-/Gelenkteilen 8 befestigen zu können, können die Verbindungsmittel 16, wie in Fig. 2 und 3 dargestellt, an beiden Prothesenteilen 26, 28 vorgesehen sein. Alternativ hierzu ist es auch möglich, die Verbindungsmittel 16 an nur einem der Prothesenteile 26; 28 vorzusehen.

Darüber hinaus kann das scharnierartige Gelenk 32 nicht nur, wie in Fig. 2 und 3 angedeutet, in seitlicher Position, sondern je nach Verlauf des Nervs 4 auch in jeder anderen Winkelstellung, wie insbesondere an einer Unter- oder Oberseite des Knochens 2 angeordnet werden (nicht dargestellt).

In jedem Fall können die Prothesenteile 26, 28 aus einem massiven Material wie beispielsweise einer Titanlegierung gebildet sein. Dabei können die Prothesenteile 26, 28 bevorzugterweise eine spezielle Beschichtung, wie beispielsweise aus Hydroxilapatit, adhäsiven Zellschichten, Titanplasma oder eine gestrahlte, säuregeätzte Oberfläche aufweisen. Alternativ hierzu können die Prothesenteile 26, 28 aber auch aus einer Gitterstruktur gebildet sein, die mit einem knochenaufbauenden Material gefüllt ist, das beispielsweise Knochenpartikel aufweist.

Wie aus den Fig. 2 und 3 ferner zu entnehmen ist, können in eine Außenseite 34 der Prothese 12 ferner Implantataufnahmen 36 eingelassen sein, an denen zu einem späteren Zeitpunkt Implantate 38, wie beispielsweise Zahnkronen, Zahnbrücken oder herausnehmbarer Zahnersatz, in einfacher Weise befestigt werden können.

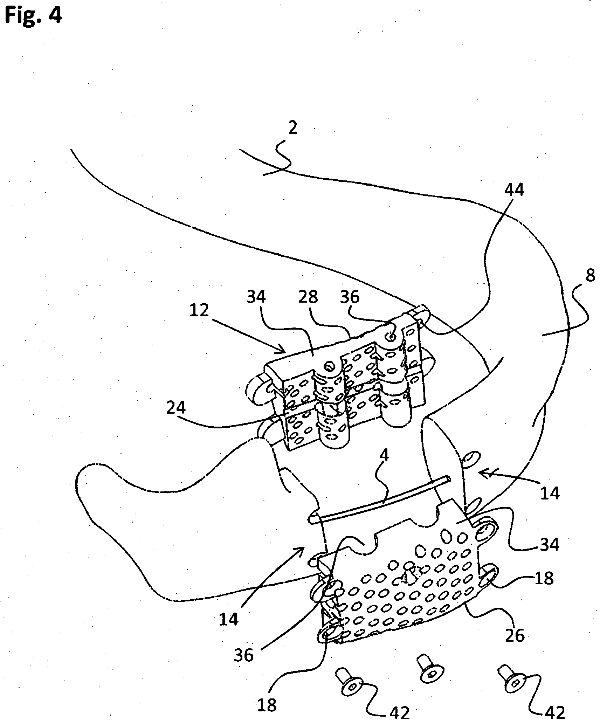

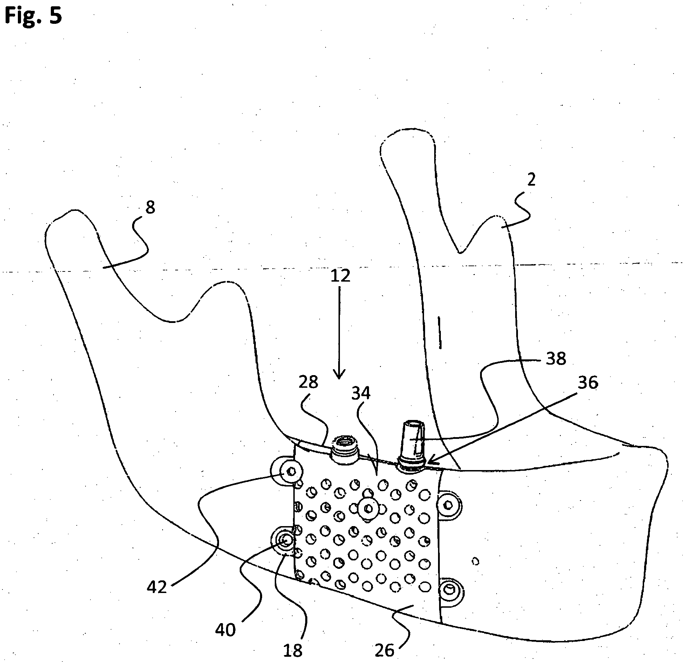

Die Fig. 4 und 5 zeigen eine alternative Ausführungsform der erfindungsgemäßen Prothese 12, bei der die beiden Prothesenteile 26, 28 voneinander getrennt ausgeführt sind und lediglich über eine Schraubverbindung aneinander festgelegt werden. In beide Prothesenteile 26, 28 sind dabei Ausnehmungen eingelassen, die sich zwischen den beiden Enden E erstecken und beim aneinander Festlegen den Aufnahmekanal 24 für den Nerv 4 bilden.

Die Festlegung erfolgt dabei unter teilweiser Zwischenlage des erhaltenen Knochen- oder Gelenkteils 8. Hierzu sind an den in den erhaltenen Knochen- oder Gelenkteil 8 eingelassenen Aufnahmekonturen 14 Aufnahmebohrungen 40 vorgesehen, in die Schrauben 42 eingeschraubt werden, die die Aufnahmeöffnungen 18 eines der beiden Prothesenteile 26; 28 durchgreifen. Die Aufnahmebohrungen 40 können dabei als Durchtrittsbohrungen ausgebildet sein, so dass die Schrauben 42 an einem jeweils entsprechend ausgebildeten Aufnahmegewinde 44 des jeweils anderen Prothesenteils 28; 26 festgeschraubt werden. Auf diese Weise fungieren die Schrauben 42 sowohl zur festen Verbindung der beiden losen Prothesenteile 26, 28 als auch zur Festlegung der gesamten Prothese 12 an dem erhaltenen Knochen- oder Gelenkteil 8.

Es ist auch möglich beidseitige Aufnahmebohrungen 40 in Form von Sacklöchern vorzusehen, an denen beide Prothesenteile 26; 28 jeweils verschraubt werden können Alternativ hierzu ist es auch möglich, lediglich einen Prothesenteil 26; 28 an dem erhaltenen Knochen- oder Gelenkteil 8 mittels der Schrauben 42 festzulegen und den jeweils anderen Prothesenteil 28; 26 lediglich mit dem ersteren Prothesenteil 26; 28 zu verschrauben.

In jedem Fall weist die Prothese 12 auch bei dieser Ausführungsform an ihrer Außenseite 34 Implantataufnahmen 36 auf, an denen Implantate 38 in einfacher Weise befestigt werden können.

## Patentansprüche

1. Prothese (12) zum wenigstens teilweisen Ersatz eines einen Nerv (4) führenden Knochens (2), wie insbesondere eines Kieferknochens,
die an zwei voneinander abgewandten Enden (E) Verbindungsmittel (16) für die Festlegung der Prothese (12) an verbleibenden Knochen- oder Gelenkteilen (8) aufweist, und
an der eine Nervaufnahme (22) für die Aufnahme des Nervs (4) vorgesehen ist, die sich zwischen den beiden Enden (E) der Prothese (12) erstreckt, wobei
die Prothese (12) einen ersten Prothesenteil (26) und einen zweiten Prothesenteil (28) aufweist und die Nervaufnahme (4) durch einen Aufnahmekanal (24) gebildet ist, der wenigstens teilweise in den ersten Prothesenteil (26) eingelassen ist, **dadurch gekennzeichnet, dass** der Aufnahmekanal durch den zweiten Prothesenteil (28) verschließbar ist, wobei in der Nervaufnahme ein viskoses Lagermittel (30) vorgesehen ist.

2. Prothese nach Anspruch 1, **dadurch gekennzeichnet, dass** der zweite Prothesenteil (28) beweglich am ersten Prothesenteil (26) gelagert ist.

3. Prothese nach Anspruch 2, **dadurch gekennzeichnet, dass** der zweite Prothesenteil (28) über ein scharnierartiges Gelenk (32) am ersten Prothesenteil (26) verschwenkbar gelagert ist.

4. Prothese nach Anspruch 2, **dadurch gekennzeichnet, dass** der zweite Prothesenteil (28) separat zum ersten Prothesenteil (26) ausgebildet und mit diesem verschraubbar ist.

5. Prothese nach einem der Ansprüche 1 bis 4, **dadurch gekennzeichnet, dass** an wenigstens einem Ende (E) eine Aufnahmeöffnung (18) zur Aufnahme eines Schraubmittels (20) vorgesehen ist.

6. Prothese nach einem der Ansprüche 1 bis 5, **dadurch gekennzeichnet, dass** in eine Außenseite (34) der Prothese (12) wenigstens eine Implantataufnahme (36) für die Festlegung eines Implantats (38) eingelassen ist.

7. Prothese nach einem der Ansprüche 1 bis 6, **dadurch gekennzeichnet, dass** die Prothese wenigstens teilweise aus einem massiven Material gebildet ist.

8. Prothese nach einem der Ansprüche 1 bis 7, **dadurch gekennzeichnet, dass** die Prothese wenigstens zum Teil aus einer Gitterstruktur gebildet ist, die mit einem knochenaufbauenden Material gefüllt ist.

## Claims

1. Prosthesis (12) for at least partial replacement of a bone (2) conveying a nerve (4), such as, in particular, a mandible bone,
comprising connecting means (16) at two ends (E) facing away from each other, for the securing of the prosthesis (12) to remaining parts (8) of bones or joints, and
in which a nerve accommodation space (22) for accommodating the nerve (4) is provided, extending between the two ends (E) of the prosthesis (12), wherein
the prosthesis (12) comprises a first prosthesis part (26) and a second prosthesis part (28), and the nerve accommodation space (22) is formed by an accommodation channel (24) which is at least partially embedded into the first prosthesis part (26), **characterized in that** the accommodation channel can be closed by means of the second prosthesis part (28), wherein a viscous bearing means (30) is provided in the nerve accommodation space.

2. Prosthesis according to claim 1, **characterized in that** the second prosthesis part (28) is movably mounted on the first prosthesis part (26).

3. Prosthesis according to claim 2, **characterized in that** the second prosthesis part (28) is pivotably mounted on the first prosthesis part (26) by means of a hinge-type joint (32).

4. Prosthesis according to claim 2, **characterized in that** the second prosthesis part (28) is formed separately from the first prosthesis part (26) and can be screwed to it.

5. Prosthesis according to any one of claims 1 to 4, **characterized in that** an accommodation opening (18) is provided at least at one end (E) in order to accommodate screw means (20).

6. Prosthesis according to any one of claims 1 to 5, **characterized in that** at least one implant accommodation mounting (36) for the securing of an implant (38) is let into an outer side (34) of the prosthesis (12).

7. Prosthesis according to any one of claims 1 to 6, **characterized in that** the prosthesis is formed at least partially from a solid material.

8. Prosthesis according to any one of claims 1 to 7, **characterized in that** the prosthesis is formed at least partially from a lattice structure, which is filled with a bone build-up material.

## Revendications

1. Prothèse (12) pour remplacer au moins partiellement un os (2), en particulier un os de mâchoire, conduisant un nerf (4),
présentant en deux extrémités (E) opposées l'une à l'autre des moyens de raccordement (16) pour la fixation de la prothèse (12) sur des parties restantes d'os ou d'articulation (8), et
sur laquelle, un logement de nerf (22) est prévu pour loger le nerf (4), logement qui s'étend entre les deux extrémités (E) de la prothèse (12),
la prothèse (12) présentant une première pièce de prothèse (26) et une deuxième pièce de prothèse (28) et le logement de nerf (22) est formé par un canal de logement (24) qui est intégré au moins partiellement dans la première pièce de prothèse (26), **caractérisée en ce que** le canal de logement est fermable par la deuxième pièce de prothèse (28), un moyen de support visqueux (30) étant prévu dans le logement de nerf.

2. Prothèse selon la revendication 1, **caractérisée en ce que** la deuxième pièce de prothèse (28) est montée mobile sur la première pièce de prothèse (26).

3. Prothèse selon la revendication 2, **caractérisée en ce que** la deuxième pièce de prothèse (28) est montée pivotante sur la première pièce de prothèse (26) par l'intermédiaire d'une articulation à charnière (32).

4. Prothèse selon la revendication 2, **caractérisée en ce que** la deuxième pièce de prothèse (28) est formée séparément de la première pièce de prothèse (26) et peut être vissée avec celle-ci.

5. Prothèse selon l'une des revendications 1 à 4, **caractérisée en ce qu**'il est prévu en au moins une extrémité (E) une ouverture de logement (18) pour loger un moyen de vissage (20).

6. Prothèse selon l'une des revendications 1 à 5, **caractérisée en ce qu**'au moins un logement d'implant (36) est intégré dans un côté extérieur (34) de la prothèse (12) pour la fixation d'un implant (38).

7. Prothèse selon l'une des revendications 1 à 6, **caractérisée en ce qu**'elle est au moins partiellement formée d'un matériau massif.

8. Prothèse selon l'une des revendications 1 à 7, **caractérisée en ce qu**'elle est au moins en partie formée d'une structure en treillis, qui est remplie d'un matériau constituant de l'os.
